# EUROPÄISCHE PATENTANMELDUNG

(11) **EP 1 238 591 A1**
(43) Veröffentlichungstag der Anmeldung: **11.09.2002**
(21) Anmeldenummer: 02003242.1
(22) Anmeldetag: 21.02.2002
(51) Int. Cl.: A23K 1/16, A23K 1/18

(54) **Bacteriocinhaltiges Sorbinsäurepräparat als Futtermittelzusatz in der Nutztieraufzucht**

(30) Priorität: 05.03.2001 DE 10110431
(71) Anmelder: Nutrinova Nutrition Specialties & Food Ingredients GmbH, 65929 Frankfurt am Main (DE)
(72) Erfinder: Raczek, Nico N., Dr., 65779 Kelkheim (DE)
(74) Vertreter: Schweitzer, Klaus, Dr.

(57) **Zusammenfassung**

Die vorliegende Erfindung betrifft ein Präparat zum Einsatz in Tierfuttermitteln. Das Präparat enthält Sorbinsäure und lebende oder tote Mikroorganismen, welche Bacteriocine ausscheiden, oder die Bacteriocine selbst oder Kombinationen daraus sowie gegebenenfalls einen Träger. Weiterhin betrifft die Erfindung die Verwendung des Präparates allein in Futtermitteln oder in Abmischung mit anderen Futtermittelzusatzstoffen zur Verbesserung des hygienischen Status des Futters und zur Leistungsförderung in der Nutztieraufzucht.

## Beschreibung

Die Erfindung betrifft ein Präparat, welches Sorbinsäure und mindestens ein Bacteriocin enthält und allein in Futtermitteln oder in Abmischung mit anderen Futtermittelzusatzstoffen in der Nutztieraufzucht angewendet werden kann.

Im Bereich der Tierfutter werden häufig Antibiotika als Leistungsförderer verwendet. Teilweise werden sehr ähnliche oder die gleichen Substanzen in der Humanmedizin verwendet. Die Anwendung von Antibiotika im Bereich der Tierernährung steht grundsätzlich im Verdacht für Gefahren verantwortlich zu sein, welche von resistenten Bakterien ausgehen, die auch die menschliche Gesundheit langfristig gefährden können. Daher müssen gesundheitlich weniger bedenkliche Produkte für diesen Einsatzzweck gesucht werden. So werden auch in anderen Bereichen zunehmend physiologisch und epidemiologisch gesundheitlich bedenkliche oder aber für die Umwelt schädliche Substanzen, wie beispielsweise Antibiotika, Formaldehyd abspaltende Stoffe, halogenierte Substanzen, u.v.a.m. beispielsweise in Lebensmitteln, Futtermitteln, Haustierfutter, Silagen, Trester, oder anderen Abfällen aus der Lebensmittelindustrie, durch weniger bedenkliche Stoffe ersetzt. Der Zweck dieser Stoffe ist zum einen auf die Werterhaltung des eigentlichen Produktes gerichtet. Zum anderen aber soll auch deren hygienischer Zustand verbessert werden, bzw. eine verlängerte Haltbarkeit erzielt werden.

Es ist bekannt, dass Sorbinsäure zur Konservierung von Futtermitteln eingesetzt werden kann. Sorbinsäure (trans,trans-2,4-Hexadiensäure) ist eine farblose, feste Verbindung, die sich nur wenig in kaltem Wasser löst und weltweit als Konservierungsstoff verwendet wird. Das Wirkprinzip wird durch die Sorbinsäure in undissoziierter Form bestimmt. Die beste Wirkung entfaltet Sorbinsäure daher im sauren pH-Bereich. Sorbinsäure und ihre Salze besitzen eine sehr gute mikrobiostatische, antimykotische Wirkung. Gleichzeitig ist Sorbinsäure als ungesättigte Fettsäure praktisch ungiftig, was sehr umfangreiche Daten belegen und was durch die jahrzehntelange Anwendung dieser Säure im Lebensmittelbereich, in Tierfuttern u.a. belegt ist.

Neben Sorbinsäure werden auch andere organische Säuren seit Jahren zur Konservierung von Futtermitteln und zur Verbesserung der Futterhygiene eingesetzt. Gerade im Futter für Jungtiere sind an die hygienische Qualität besondere Anforderungen zu stellen. Daher sind einige organischen Säuren als Futterzusatzstoffe, basierend auf den nationalen futtermittelrechtlichen Bestimmungen ohne Höchstmengenbegrenzung zugelassen.

Bacteriocine sind spezifische Hemmstoffe welche von Mikroorganismen ausgeschieden werden und letal für andere Mikroorganismen - vorwiegend Bakterien - sind. Bacteriocine sind Peptide, Polypeptide, Proteine oder Substanzen welche zumindest proteinogene Strukturen aufweisen und aus Aminosäuren zusammengesetzt sind. Dabei können diese aus Aminosäuren zusammengesetzten Bacteriocine auch ungewöhnliche Aminosäuren, wie beispielsweise Lanthionin oder β-Methyllanthionin enthalten. Beispielsweise enthält Pediocin L50 weitere modifizierte Aminosäuren (L.M. Cintas et al., "Isolation and Characterization of Pediocin L50, a New Bacteriocin from *Pediococcus acidilactici* with a Broad Inhibitory Spectrum", Applied and Environmental Microbiology, July 1995, Seiten 2643-2648).

Mikroorganismen, welche Bacteriocin produzieren, kommen häufig natürlicherweise beispielsweise in Milch und Milchprodukten vor (vgl. z.B. E. Rodriguez et al., "Diversity of bacteriocins produced by lactic acid bacteria isolated from raw milk", International Dairy Journal 10 (2000) 7-15). Auch werden solche Mikroorganismen immer wieder aus anderen Lebensmitteln wie Fleisch und Fleischprodukten isoliert (vgl. beispielsweise Food Science and Technology International (1998) 4, 141-158).

Häufig werden die Mikroorganismen welche Bacteriocine ausscheiden schon seit mehreren Jahrhunderten - häufig unwissend - zur Herstellung von Lebensmitteln derart verwendet, dass die als sogenannte Schutzkulturen absichtlich zugesetzten Bakterien durch deren Ausscheidungsprodukte andere, verderbniserregende, toxische, unerwünschte oder anderweitig gefährliche Bakterien hemmen. Ein bekanntes Bacteriocin ist Nisin. Dieses wird kommerziell hergestellt und gegen bestimmte Mikroorganismen welche die sogenannte "Spätblähung" im Käse verursachen seit Jahren auch in Lebensmittel als Zusatzstoff eingesetzt.

Grundsätzlich nachteilig an der Verwendung der Bacteriocine ist, dass sie nur gegen bestimmte Mikroorganismengruppen, insbesondere gegen nahe Verwandte, wirken. Außerdem sind Bacteriocine instabil im Lebensmittel und zerfallen nach gewisser Zeit, so dass keine Wirksamkeit mehr erreicht werden kann.

Bei den bekannten anderen organischen Säuren als Futtermittelzusatz wirkt sich nachteilig aus, dass diese zum Teil flüchtig sind, unangenehm riechen und darüber hinaus korrosiv wirken. Die mit ihnen erreichbaren leistungsfördernden Effekte gehen mit erheblichen Nachteilen in der Handhabung einher.

Es bestand demnach die Aufgabe, einen einfach zu handhabenden konservierend wirkenden, leistungsfördernden stabilen Zusatz zur Verfügung zu stellen, der diese Nachteile nicht aufweist.

Gelöst wird diese Aufgabe durch ein Präparat (Zusammensetzung), welches Sorbinsäure sowie mindestens ein Bacteriocin enthält. Das oder die Bacteriocine werden als solche eingesetzt; es ist aber auch durchaus möglich, lebende oder tote Mikroorganismen einzusetzen, die diese Bacteriocine produzieren oder enthalten. Vorzugsweise werden bacteriocinproduzierende Mikroorganismen verwendet, welche natürlich vorkommen, beispielsweise in Milch- oder Fleischprodukten. Erfindungsgemäß einzusetzende Mikroorganismen sind nur solche, die Bacteriocine produzieren. In der unten aufgeführten Tabelle sind Spezien von Mikroorganismen enthalten, die sowohl Bacteriocine produzieren können als auch nicht (bspw. Bacillus cereus); einsetzbar sind diese dementsprechend nur dann, wenn sie Bacteriocine produzieren. Die Bacteriocin produzierenden bzw. enthaltenden Mikroorganismen oder die Bacteriocine selbst können auch in verkapselter Form oder auf Trägern gebunden eingesetzt werden. Weiterhin können Präparate verwendet werden, welche Bacteriocine in noch wirksamen Konzentrationen oder nachweisbaren Mengen enthalten. Dies schließt auch Abmischungen solcher Präparate, beispielsweise mit Molkeproteinen oder Kochsalz mit ein. Gängige Produkte dieser Art sind z.B. ALTA2341 (Quest Biotechnology, Inc., Sarasota, U.S.A.) Microgard (Rhône Poulenc, Courbevois, Frankreich).

Bevorzugt werden die in der folgenden Tabelle genannten Bacteriocine/Mikroorganismen eingesetzt.

| **Mikroorganismen** | **Bacteriocine** |
|---|---|
| *Aeromonas hydrophila Lactobacillus sakei* | Sakacin A oder P |
| *Bacillus cereus* | Lactocin-S, Lactostrepcin-5, Pediocin-A, Pediocin-AcH, Sakacin-A |
| *Bacillus coagulans Bacillus licheniformis Bacillus stearothermophilus Clostridium bifermentans* | Nisin |
| *Lactococcus lactis* | |
| *Bacillus pumilis* | Thermophillin |
| *Bacillus subtilis, 168, JH642* | Subtilin, Lacticin-481, Nisin, Thermophillin, Subtilosin |
| *Bronchothrix thermospacta* | Curvacin-A, Pediocin-AcH, Sakacin-A, Sakacin-P |
| *Camobacterium divergens* | |
| *Camobacterium piscicola UI49, LV 17 oder LV 61* | Carnocin UI 49, Carnobacteriocin A, B1 und B2; Piscicolin 61 |
| *Clostridium botulinum* | Nisin, Pediocin-A, Reuterin, Sakacin-A |
| *Clostridium butyricum* | Nisin, Reuterin |
| *Clostridium perfringens* | Nisin, Pediocin-A, Pediocin-AcH, Pediocin-VTT, Reuterin, Thermophillin |
| *Clostridium sporogens* | Nisin, Pediocin-A |
| *Clostridium tyrobutricum* | Lacticin-481, Lactocin-S, Pediocin-AcH |
| *Enterococcus faecalis* | |
| *Enterococcus faecalis 226, INIA 4* | Enterocin 226NWC, AS-48 |
| *Enterococcus faecalis S-48* | Bacteriocin Bc-48 |
| *Enterococcus faecium, BFE 900, CTC492, cal 1, NIAI157, A, B, P, L 50, G 16, AA13, T136* | Enterocin 1146, B, A, Cal, ON- 157, P, L50A, L50B |
| *Enterococcus spp.* | Enterococcins (I-V) |
| *Escherichia coli* | Reuterin, Thermophillin |
| *Fusobacterium mortiferum, (z.B.: "FM1025")* | |
| *Lactobacillus acidophilus* | Lactocicin |
| *Lactobacillus acidophilus 11088, OSU 133, 2181, DDS1, LAPT, 1060, M46, N2, TK8912*, | Lactacin F, Lacidin, Acidolin, Acidophillin, Acidophilucin A, Bacteriocin M46, Lactacin B, Acidocin 8912, Lactacin B |
| *Lactobacillus amylovorus DCE 471* | Amylovorin L471 |
| *Lactobacillus bavaricus MI401* | Bavaricin A |
| *Lactobacillus bulgaricus* | Bulgarican |
| *Lactobacillus brevis* | Lactobacillin |
| *Lactobacillus brevis* | Brevicin |
| *Lactobacillus casei B80 Lactobacillus casei LHS* | Caseicin 80, Caseicin LHS |
| *Lactobacillus curvatus LTH 1174, SB 13* | Curvacin A, 13 |
| *Lactobacillus delbrückii ssp. bulgaricus* | Bulgarican |
| *Lactobacillus delbrueckii subsp. lactis JCM 1106, JCM 1107, JCM 1248* | Lacticin B |
| *Lactobacillus fermentum466* | Bacteriocin 446, Proteid |
| *Lactobacillus gasseri* | Gassericin A |
| *Lactobacillus helveticus* | Lactocin 27 |
| *Lactobacillus helveticus 1829, 481, LP27* | Helveticin V-1829, Helveticin J, Lactocin 27 |
| *Lactobacillus plantarum, A2, BN, C-11, LPCO-10, LPCO-10, MI406, NCDO 1193, SIK-83, 35 d, CTC 305,* | Plantaricin A und D, Lactolin, Plantaricin BN, A, S, 406, -B, SIK-83, 35 d |
| *Lactobacillus reuteri LA6* | Reutericin 6 |
| *Lactobacillus sakei, Lb 706, L45, LTH 673, CTC 494,* CTC 372, *148* | Sakacin-A, Lactocin S, Sakacin P, Sakacin K und T |
| *Lactococcus lactis subsp. cremoris, - 202, - 9B4, - 346, - 9B4, 4G6, LMG 2130, LMG2081, JW3* | Diplococcin, Lactostrepcin 5, Lactococcin A, B und M, Bac I, II, III und IV, Lactococcin A, G, Lacticin |
| *Lactococcus lactis subsp. lactis 10, 300, 71, ADRIA 85LO30, CNRZ 481, DRC1, 6F3* | Lactostrepcin 1, 2,3, 4 und DR, Lacticin 481, Dricin, Bac V, VI und VII |
| *Lactococcus lactis subsp. lactis ATCC 11454* | Nisin A |
| *Lactococcus lactis subsp. lactis NIZO 22186* | Nisin Z |
| *Lactococcus lactis subsp. lactis var. diacetylctis 6F7* | Bac VIII |
| *Lactococcus lactis subsp. lactis var. diacetylctis DPC938, S50 und WM4* | Lactocin D, Bacteriocin S50, Bac WM4 |
| *Leuconostoc carnosum z.B.: Lm1* | Leucococin Lcm1 |
| *Leuconostoc dextranicum* | |
| *Leuconostoc geldium z.B.: UAL 187* | Leucocin A-UAL 187 |
| *Leuconostoc gelidium* | |
| *Leuconostoc mesenteroides* | |
| *Leuconostoc mesenteroides subsp. mesenteroides FR52, UL5, Y105* | Mesenterocin 52, 5, Y105 |
| *Leuconostoc paramesenteroides OX* | Leuconocin S |
| *Listeria innocua* | Lacticin-481, Lactosin-S, Pediocin-A, Pediocin-AcH |
| *Listeria ivanovii* | Pediocin-A, Pediocin-AcH, Pediocin-PAC10 |
| *Listeria monocytogenes spp.* | Carnobacteriocin A&B, Curvacin-A, Enterocin-1146, Lactacin-B, Lacticin-481, Leucocin-A, Nisin, Pediocin-A, Pediocin AcH, Pediocin-JD, Pediocin-PA-1, Pediocin-PAC10, Pediocin-VVT, Piscicolin-61, Reuterin, Sakacin-A, Sakacin-P |
| *Listeria seeligeri* | Pediocin-A |
| *Listeria welchii* | Lacticin-481, Pediocin-A |
| *Mycobacterium tuberculosis* | Nisin |
| *Pediococcus acidilactic e.a. H, E, F, M* | Pediocin AcH |
| *Pediococcus acidilactic JD1-23, PAC 1.0, SJ-1,* | Pediocin JD, PA-1, SJ-1 |
| *Pediococcus pentosaceus FBB-61, L-7230, N5p* | Pediocin A, N5p |
| *Proteus mirabillis* | Nisin |
| *Pseudomonas aeruginosa* | Thermophillin |
| *Pseudomonas fluorescens* | |
| *Salmonella enteritidis* | Reuterin, Thermophillin |
| *Salmonella infantis* | Pseudiocin-VVT, Reuterin |
| *Salmonella typhimurium* | Reuterin, Thermophillin |
| *Shigella sp.* | Reuterin, Thermophillin |
| *Staphylococcus aureus* | Nisin, Lacticin-481, Pediocin-A, Pediocin-AcH, Plantarcin-SIK83, Sakacin-A, Thermophillin |
| *Staphylococcus camosus* | Curvacin, Lacticin-481, Lactocin-S, Pediocin-AcH |
| *Staphylococcus epidermidis* | Nisin |
| *Staphylococcus simulans* | Nisin |
| *Streptococcus thermophilus Sfi13, St10, STB40, STB78* | Thermophillin 13, Bacteriocin St10, Bacteriocin STB40, Bacteriocin STB78 |
| *Yersinia enterocolitica* | Thermophillin |

Die Bacteriocine werden nach bekannten Verfahren gewonnen, beispielsweise durch einfache Fällung mittels Ammoniumsulfat, Gelfiltration (Sephadex G-50), Kationenaustausch chromatographie(CM-cellulose), RP-HPLC, adsorption/desorptionzentrifugieren, Vortex flow filtration oder andere technisch sinnvolle Methoden (s. Parente E. und Ricciardi A., Appl. Microbiol. Biotechnol. 1999, 52, 628-638).

Das erfindungsgemäße Präparat enthält 90,00 bis 99,90 Gew.-%, bevorzugt 95,00 bis 99,99 Gew.-% Sorbinsäure. Gewichtsprozente beziehen sich dabei auf die Gesamtmasse des Präparates.

Das bzw. die Bacteriocine sind in den erfindungsgemäßen Präparaten zweckmäßigerweise in solchen Mengen enthalten, dass im Tierfutter 2,5 bis 50 mg/kg, bevorzugt 5 bis 40 mg/kg, insbesondere 10 bis 20 mg/kg vorhanden sind. Zubereitungen, welche Bacteriocine enthalten, werden in entsprechend höherer Dosierung zugesetzt (enthält beispielsweise die Zubereitung 2,5% Bacteriocin als wirksame Substanz so werden hiervon bevorzugt 400 bis 800 mg/kg eingesetzt). Werden bacteriocinbildende Mikroorganismen oder Kombinationen derselben in den erfindungsgemäßen Präparaten eingesetzt, so sind diese bevorzugt in Mengen vorhanden, die etwa 10⁶ bis 10¹⁰ Mikroorganismen je g Futtermittel entsprechen. Es können dazu auch sprühgetrocknete Produkte verwendet werden. Dabei sollte ebenfalls im Tierfutter ein Gehalt von 2,5 bis 50 mg/kg, bevorzugt 5 bis 40 mg/kg, insbesondere 10 bis 20 mg/kg Bacteriocin erreicht werden.

Als Träger sowohl für die Sorbinsäure als auch für das Bacteriocin oder die Mikroorganismen können organische oder anorganische Materialien verwendet werden. Dazu zählen beispielsweise Stärke und andere Polysaccharide wie Cellulose. Zur besseren Verteilung in Abmischungen mit Sorbinsäure können die Bacteriocine auch in Salzen wie Kochsalz oder Mineralsalzen oder aber Molkepulver oder anderen Milchverarbeitungsprodukten vorgemischt sein.

Weiterhin können die Bacteriocine oder die Mikroorganismen mit Mikrokapseln/Mikrospheren versehen sein, um damit unerwünschten Einflüssen durch Verdauungssäfte zu widerstehen. Hierbei ist es möglich die Sorbinsäure getrennt von den Bacteriocinen in die Mikrosphere oder aber in eine der äußeren Hüllschichten einer Mikrokapsel so unterzubringen, dass Sorbinsäure früher abgelöst wird und beispielsweise im Magen bereits zu einer merklichen pH-Absenkung führt, die Bacteriocine aber erst später im Magen-Darm-Trakt freigesetzt werden. Auch eine Mischung von verkapselten Bacteriocinen und Sorbinsäure ist möglich. Für die Verkapselung eignet sich beispielsweise Gelatine, Lecithine, Stearate, Alginate, Traganth, Xanthan, Carrageen, Cassia-Gum, Gummi arabicum, Maltodextrine, modifizierte Stärken, Cellulosen, Monound Diglyceride von Speisefettsäuren, verestert mit organischen Säuren oder nicht verestert, feste Triglyceride mit vorzugsweise gesättigten Fettsäuren wie Tripalmitin, feste Fettsäuren wie Palmitinsäure oder Mischungen daraus.

Als Träger und zur Stabilisierung der Präparate werden >0 bis 10 Gew.-%, bevorzugt 2,5 bis 7,5 Gew.-% (bezogen auf das Präparat) Trägermaterialien allein oder in Kombination eingesetzt.

Das erfindungsgemäße Präparat wird hergestellt, indem man beispielsweise die Sorbinsäure und Bacteriocine, Bacteriocinmischungen, Zubereitungen, welche Bacteriocine enthalten, oder lebenden oder abgestorbene Mikroorganismen, welche Bacteriocine gebildet haben, mechanisch durchmischt. Enthält das erfindungsgemäße Präparat einen Träger, so werden zweckmäßigerweise in einem handelsüblichen Taumel- oder anderen üblichen Mischer zunächst die u.U. flüssige(n) Mikroorganismenextrakte auf den Träger aufgebracht und anschließend die Sorbinsäure und die anderen festen Bestandteile zugegeben.

Als Tierfuttermittel geeignet sind z. B. Grünfutter, Silagen, Trockengrünfutter, Wurzeln, Knollen, fleischige Früchte, Körner und Samen, Biertreber, Trester, Bierhefe, Destillationsschlempe, Müllereinebenerzeugnisse, Nebenerzeugnisse der Zucker-, Stärkeherstellung und Ölgewinnung und verschiedene Lebensmittelabfälle. Solchen Futtermitteln können bestimmte Futtermittelzusatzstoffe (z.B. Antioxidantien) oder Mischungen aus verschiedenen Substanzen (z.B. Mineralstoffmischungen, Vitaminmischungen) zu Verbesserung beigegeben werden. Spezielle Futtermittel werden auch für bestimmte Tierarten und deren Entwicklungsstadium angepasst. Dies ist beispielsweise in der Ferkelaufzucht der Fall. Hier werden Saugferkelfutter und Ferkelaufzuchtfutter verwendet. Das erfindungsgemäße Präparat kann direkt dem Tierfuttermittel bzw. auch in Abmischung mit anderen Futtermittelzusatzstoffen oder aber über Vormischungen dem eigentlichen Futtermittel zugegeben werden. Das Präparat kann trocken dem Futter zugemischt werden, vor einer weiteren Verarbeitung (z.B. Extrusion) zugegeben werden bzw. im Gemisch dispergiert zudosiert werden. Darüber hinaus können auch die Einzelbestandteile des Präparats getrennt einzelnen Bestandteilen des Futtermittels zugegeben werden. Für diese Zwecke werden zweckmäßigerweise Präparat-Konzentrationen zwischen 0,25 und 7,5 Gew.-% (bezogen auf das Futter), bevorzugt 0,75 bis 4,0 Gew.-% angewendet.

Das Präparat kann als alleiniger Zusatzstoff zu Tierfuttermitteln beispielsweise für die Rinder-, Geflügel-, Kaninchen- oder Schafaufzucht, besonders bevorzugt zu Saugferkelfutter (Prestarter) und Ferkelaufzuchtfutter (Starterfutter), zugegeben werden oder in Abmischung mit anderen Futtermittelzusatzstoffen für diese Tiere angewendet werden. Weiterhin eignen sich Futtermittel mit dem erfindungsgemäßen Präparat als Milchaustauscher bei der Frühentwöhnung von Sauglämmern oder Kälbern.

Überraschenderweise zeigen die erfindungsgemäßen Präparate die oben beschriebenen Nachteile nicht. Die Präparate zeigen vielmehr gute Eigenschaften in der Handhabung. Außerdem wird eine effektive Säuerung des Futters erreicht. Zudem kann überraschenderweise auch ein positiver Einfluss auf die Wachstumsleistung von jungen Tieren schon in relativ geringen Präparatemengen festgestellt werden.

Die erfindungsgemäßen Präparate liegen in einem festen Aggregatzustand vor. Durch die vorliegende Erfindung wird die sonst problematische Handhabbarkeit der bisher verwendeten flüssigen Säuren vermieden. Weiterhin ist das erfindungsgemäße Präparat in der Lage den hygienischen Status zu verbessern, dadurch dass unerwünschte Organismen und Verderbserreger unterdrückt werden, die anderenfalls vorhandene Nährstoffe verbrauchen können.

Überraschenderweise wurde gefunden, dass schon durch Zusatz geringer Mengen erfindungsgemäßer Präparate in der Ferkelaufzucht eine deutliche Leistungsverbesserung hinsichtlich Zuwachsrate und Futterverwertung erreicht werden kann. Zur Sicherung einer signifikanten nutritiven Wirksamkeit ist ein Zusatz an erfindungsgemäßen Präparaten in Mengen von 0,25 bis 7,5 Gew.-%, bezogen auf das Futter, vorzugsweise von 0,75 bis 4,0 Gew.-%, zweckmäßig.

Die Erfindung wird nachfolgend anhand von Beispielen erläutert.

### Beispiel 1

0,0075 bis 0,015 kg (entsprechend einer Konzentration von mindestens 20 mg/kg Bacteriocin in dem Futter) eines mit Molkenpulver versprühten, getrockneten, mit Bacteriocinen angereicherten Präparates aus *Lactococcus lactis subsp. cremoris* und *Lactobacillus plantarum* werden mit 1,0 kg Sorbinsäure in einem Doppelkonusmischer mit Taumelbewegungen über eine Zeitdauer von etwa 15 min gemischt. Die homogene Mischung wird mit 100 kg Ferkelfutter folgender Zusammensetzung vermischt (folgende Angaben in Gew.-%).

| | |
|---|---|
| Fischmehl | 4,00 |
| Sojaextraktionsschrot | 18,50 |
| Gerste | 40,00 |
| Weizen | 33,00 |
| Pflanzenöl | 1,90 |
| L-Lysin - HCI | 0,2 |
| DL-Methionin | 0,1 |
| L-Threonin | 0,1 |
| Mineralfutter | 2,2 |

### Beispiel 2

0,08 kg einer Abmischung von Nisin (Nisaplin Aplin & Barrett, Dorset, U.K.) mit Molkeproteinen und Kochsalz, welche 2,5 Prozent Reinsubstanz (entspricht etwa 1 x 10⁶ IU/g oder 1 x 10⁶ Reading units/g) enthält, werden mit 0,92 kg Sorbinsäure in einem Doppelkonusmischer mit Taumelbewegungen über eine Zeitdauer von etwa 15 min gemischt, so dass eine gleichmäßige Vermischung erreicht wird. Diese Mischung wird mit 100 kg Ferkelfutter folgender Zusammensetzung vermischt (folgende Angaben in Gew.-%).

| | |
|---|---|
| Sojaextraktionsschrot | 22,00 |
| Gerste | 40,00 |
| Weizen | 31,00 |
| Pflanzenöl | 2,90 |
| L-Lysin - HCI | 0,40 |
| DL-Methionin | 0,10 |
| L-Threonin | 0,10 |
| Mineralfutter | 3,50 |

Es wurde gefunden, dass schon durch Zusatz dieser Mengen erfindungsgemäßer Präparate in der Ferkelaufzucht eine deutliche Leistungsverbesserung hinsichtlich Zuwachsrate und Futterverwertung erreicht wird.

## Patentansprüche

1. Präparat, welches Sorbinsäure und mindestens ein Bacteriocin enthält.

2. Präparat nach Anspruch 1, **dadurch gekennzeichnet, dass** das Präparat 90,00 bis 99,90 Gew.-% Sorbinsäure enthält.

3. Präparat nach Anspruch 1 oder 2, **dadurch gekennzeichnet, dass** die Konzentration des Bacteriocins oder der Bacteriocine so bemessen ist, dass in dem Tierfutter, in welchem das Präparat eingesetzt wird, 2,5 bis 50 mg/kg mindestens eines Bacteriocins vorhanden ist.

4. Präparat nach einem der Ansprüche 1 bis 3, **dadurch gekennzeichnet, dass** das Bacteriocin oder ein bacteriocinproduzierender Mikroorganismus ausgewählt wird aus einer oder mehreren der folgenden Substanzen:
| **Mikroorganismen** | **Bacteriocine** |
|---|---|
| *Aeromonas hydrophila Lactobacillus sakei* | Sakacin A oder P |
| *Bacillus cereus* | Lactocin-S, Lactostrepcin-5, Pediocin-A, Pediocin-AcH, Sakacin-A |
| *Bacillus coagulans Bacillus licheniformis Bacillus stearothermophilus Clostridium bifermentans Lactococcus lactis* | Nisin |
| *Bacillus pumilis* | Thermophillin |
| *Bacillus subtilis, 168, JH642* | Subtilin, Lacticin-481, Nisin, Thermophillin, Subtilosin |
| *Bronchothrix thermospacta* | Curvacin-A, Pediocin-AcH, Sakacin-A, Sakacin-P |
| *Camobacterium divergens* | |
| *Camobacterium piscicola UI 49, LV 17 oder LV 61* | Carnocin UI 49, Carnobacteriocin A, B1 und B2; Piscicolin 61 |
| *Clostridium botulinum* | Nisin, Pediocin-A, Reuterin, Sakacin-A |
| *Clostridium butyricum* | Nisin, Reuterin |
| *Clostridium perfringens* | Nisin, Pediocin-A, Pediocin-AcH, Pediocin-VTT, Reuterin, Thermophillin |
| *Clostridium sporogens* | Nisin, Pediocin-A |
| *Clostridium tyrobutricum* | Lacticin-481, Lactocin-S, Pediocin-AcH |
| *Enterococcus faecalis* | |
| *Enterococcus faecalis 226, INIA 4* | Enterocin 226NWC, AS-48 |
| *Enterococcus faecalis S-48* | Bacteriocin Bc-48 |
| *Enterococcus faecium, BFE 900, CTC492, cal 1, NIAI157, A, B, P, L 50, G 16, AA13, T136* | Enterocin 1146, B, A, Cal, ON- 157, P, L50A, L50B |
| *Enterococcus spp*. | Enterococcins (I-V) |
| *Escherichia coli* | Reuterin, Thermophillin |
| *Fusobacterium mortiferum*, *(z.B.: "FM1025")* | |
| *Lactobacillus acidophilus* | Lactocicin |
| *Lactobacillus acidophilus 11088, OSU 133, 2181, DDS1, LAPT, 1060, M46, N2, TK8912,* | Lactacin F, Lacidin, Acidolin, Acidophillin, Acidophilucin A, Bacteriocin M46, Lactacin B, Acidocin 8912, Lactacin B |
| *Lactobacillus amylovorus DCE 471* | Amylovorin L471 |
| *Lactobacillus bavaricus MI401* | Bavaricin A |
| *Lactobacillus bulgaricus* | Bulgarican |
| *Lactobacillus brevis* | Lactobacillin |
| *Lactobacillus brevis* | Brevicin |
| *Lactobacillus casei B80 Lactobacillus casei LHS* | Caseicin 80, Caseicin LHS |
| *Lactobacillus curvatus LTH 1174, SB 13* | Curvacin A, 13 |
| *Lactobacillus delbrückii ssp. bulgaricus* | Bulgarican |
| *Lactobacillus delbrueckii subsp. lactis JCM 1106, JCM 1107, JCM 1248* | Lacticin B |
| *Lactobacillus fermentum466* | Bacteriocin 446, Proteid |
| *Lactobacillus gasseri* | Gassericin A |
| *Lactobacillus helveticus* | Lactocin 27 |
| *Lactobacillus helveticus 1829, 481, LP27* | Helveticin V-1829, Helveticin J, Lactocin 27 |
| *Lactobacillus plantarum, A2, BN, C-11, LPCO -10, LPCO -10, MI406, NCDO 1193, SIK-83, 35 d, CTC 305,* | Plantaricin A und D, Lactolin, Plantaricin BN, A, S, 406, -B, SIK-83, 35 d |
| *Lactobacillus reuteri LA6* | Reutericin 6 |
| *Lactobacillus sakei, Lb 706, L45, LTH 673, CTC 494, CTC 372, 148* | Sakacin-A, Lactocin S, Sakacin P, Sakacin K und T |
| *Lactococcus lactis subsp. cremoris, - 202, - 9B4, - 346, - 9B4, 4G6, LMG 2130, LMG2081, JW 3* | Diplococcin, Lactostrepcin 5, Lactococcin A, B und M, Bac I, II, III und IV, Lactococcin A, G, Lacticin |
| *Lactococcus lactis subsp. lactis 10, 300, 71, ADRIA 85LO30*, *CNRZ 481, DRC1, 6F3* | Lactostrepcin 1, 2,3, 4 und DR, Lacticin 481, Dricin, Bac V, VI und VII |
| *Lactococcus lactis subsp. lactis ATCC 11454* | Nisin A |
| *Lactococcus lactis subsp. lactis NIZO 22186* | Nisin Z |
| *Lactococcus lactis subsp. lactis var. diacetylctis 6F7* | Bac VIII |
| *Lactococcus lactis subsp. lactis var. diacetylctis DPC938, S50 und WM4* | Lactocin D, Bacteriocin S50, Bac WM4 |
| *Leuconostoc carnosum z.B.: Lm1* | Leucococin Lcm1 |
| *Leuconostoc dextranicum* | |
| *Leuconostoc geldium z.B.: UAL 187* | Leucocin A-UAL 187 |
| *Leuconostoc gelidium* | |
| *Leuconostoc mesenteroides* | |
| *Leuconostoc mesenteroides subsp. mesenteroides FR52, UL5, Y105* | Mesenterocin 52, 5, Y105 |
| *Leuconostoc paramesenteroides* OX | Leuconocin S |
| *Listeria innocua* | Lacticin-481, Lactosin-S, Pediocin-A, Pediocin-AcH |
| *Listeria ivanovii* | Pediocin-A, Pediocin-AcH, Pediocin-PAC10 |
| *Listeria monocytogenes spp.* | Carnobacteriocin A&B, Curvacin-A, Enterocin-1146, Lactacin-B, Lacticin-481, Leucocin-A, Nisin, Pediocin-A, Pediocin AcH, Pediocin-JD, Pediocin-PA-1, Pediocin-PAC10, Pediocin-VVT, Piscicolin-61, Reuterin, Sakacin-A, Sakacin-P |
| *Listeria seeligeri* | Pediocin-A |
| *Listeria welchii* | Lacticin-481, Pediocin-A |
| *Mycobacterium tuberculosis* | Nisin |
| *Pediococcus acidilactic e.a. H, E, F, M* | Pediocin AcH |
| *Pediococcus acidilactic JD1-23, PAC 1.0, SJ-1,* | Pediocin JD, PA-1, SJ-1 |
| *Pediococcus pentosaceus FBB-61, L-7230, N5p* | Pediocin A, N5p |
| *Proteus mirabillis* | Nisin |
| *Pseudomonas aeruginosa* | Thermophillin |
| *Pseudomonas fluorescens* | |
| *Salmonella enteritidis* | Reuterin, Thermophillin |
| *Salmonella infantis* | Pseudiocin-VVT, Reuterin |
| *Salmonella typhimurium* | Reuterin, Thermophillin |
| *Shigella sp.* | Reuterin, Thermophillin |
| *Staphylococcus aureus* | Nisin, Lacticin-481, Pediocin-A, Pediocin-AcH, Plantarcin-SIK83, Sakacin-A, Thermophillin |
| *Staphylococcus carnosus* | Curvacin, Lacticin-481, Lactocin-S, Pediocin-AcH |
| *Staphylococcus epidermidis* | Nisin |
| *Staphylococcus simulans* | Nisin |
| *Streptococcus thermophilus Sfi13, St10, STB40, STB78* | Thermophillin 13, Bacteriocin St10, Bacteriocin STB40, Bacteriocin STB78 |
| *Yersinia enterocolitica* | Thermophillin |

5. Futtermittel, **dadurch gekennzeichnet, dass** es ein Präparat nach Anspruch 1 enthält.

6. Futtermittelzusatz, **dadurch gekennzeichnet, dass** es ein Präparat nach Anspruch 1 enthält.

7. Futtermittel nach Anspruch 5, **dadurch gekennzeichnet, dass** es 0,25 bis 7,5 Gew.-%, bezogen auf das Gewicht des Futtermittels, des Präparates enthält.

8. Verwendung des Präparates nach Anspruch 1 als Tierfutter- oder Futtermittelzusatz.

9. Verwendung nach Anspruch 8 in der Schweineaufzucht.

10. Verwendung nach Anspruch 8 in der Rinderaufzucht.

11. Verwendung nach Anspruch 8 in der Lämmeraufzucht.

12. Verwendung nach Anspruch 8 in der Geflügelaufzucht.

13. Verwendung nach Anspruch 8 in der Kaninchenaufzucht.
